# EUROPÄISCHE PATENTSCHRIFT

(11) **EP 2 283 795 B1**
(45) Veröffentlichungstag und Bekanntmachung des Hinweises auf die Patenterteilung: **05.06.2013**
(21) Anmeldenummer: 10007783.3
(22) Anmeldetag: 27.07.2010
(51) Int. Cl.: A61F 5/01, A61F 5/02

(54) **Orthopädisches Stützmittel und Herstellungsverfahren hierzu**
Orthopaedic aid and corresponding production method
Moyen de support orthopédique et son procédé de fabrication

(30) Priorität: 10.08.2009 DE 102009037823
(43) Veröffentlichungstag der Anmeldung: 16.02.2011
(73) Patentinhaber: Peter Müller GmbH, 72461 Albstadt (DE)
(72) Erfinder: Müller, Markus, 72461 Albstadt (DE)
(74) Vertreter: Thum, Bernhard

(56) Entgegenhaltungen:
- WO-A1-2007/129950
- US-A- 5 185 000
- US-A- 6 142 967
- US-A1- 2008 294 082
- US-A1- 2009 156 973

## Beschreibung

### Anwendungsgebiet und Stand der Technik

Die Erfindung betrifft ein orthopädisches Stützmittel, insbesondere in Form einer Bandage, mit einem formflexiblen als Maschenware ausgebildeten und zumindest in elastischen Teilbereichen elastischen Hauptkörper, der dafür ausgebildet ist, ein zu stützendes Körperteil eines Patienten zu umgeben. Die Erfindung betrifft weiterhin auch ein Verfahren zur Herstellung eines solchen orthopädischen Stützmittels.

Gattungsgemäße orthopädische Stützmittel sind allgemein bekannt. Sie dienen beispielsweise als Sportbandagen dazu, eine präventive Stabilisierungsfunktion auszuüben und Körperteile, insbesondere Gelenke wie ein Sprunggelenk oder ein Kniegelenk, vor Schädigungen zu stützen. Erfindungsgemäße Stützmittel sind insbesondere auch dafür vorgesehen, nach bereits eingetretenen Verletzungen oder Überbeanspruchungen eines Körperteils, insbesondere eines Gelenks, Korrekturfunktionen bzw. Kompressionsfunktionen auszuüben, durch die das entsprechende Körperteil in einer orthopädisch zweckmäßigen und der Rekonvaleszenz zuträglichen Weise unterstützt und geführt wird.

Gattungsgemäße Stützmittel weisen einen als einlagige oder mehrlagige Maschenware ausgebildet Hauptkörper auf. Dabei werden nur jene Maschenwarenteile des Stützmittels dem Hauptkörper zugeordnet, die sich über die gesamte Fläche des Hauptkörpers erstrecken und die nicht auf diesem nachträglich angenäht oder anderweitig befestigt wurden. Der Hauptkörper ist zumindest im Bereich der elastischen Teilbereiche elastisch ausgebildet, um das orthopädische Stützmittel einfach anlegen zu können und um das entsprechende von dem Stützmittel umgebene Körperteil trotz des Stützmittels bewegen zu können.

Die bestimmungsgemäßen Kompressions-, Korrektur- und Stabilisierungsfunktionen stehen jedoch in einem gewissen Maße im Widerspruch zu dieser elastischen Ausgestaltung des Hauptkörpers des Stützmittels. Die Elastizität führt unerwünschter Weise auch dazu, dass zum Teil orthopädisch ungünstige Bewegungsabläufe im angelegten Zustand des orthopädischen Stützmittels möglich sind und die orthopädische Stützwirkung und Stabilisierungswirkung somit nicht ausreichend erzielt wird.

Es ist daher zweckmäßig, dem Hauptkörper des orthopädischen Stützmittels partiell und in Hinblick auf vorgegebene Richtungen die Elastizität zu nehmen. Hierzu ist es aus dem Stand der Technik bekannt, nach der Herstellung des Hauptkörpers des orthopädischen Stützmittels Zugbänder oder ähnliches am Hauptkörper anzubringen, die nach Anlegen des orthopädischen Stützmittels gezielt dessen Elastizität einschränken. Nachteilig an einer solchen Lösung mit nachträglich außenseitig angebrachten Zugbändern sind der Herstellungsaufwand und die erhebliche Dicke, die der Hauptkörper eines derartig ausgebildeten Stützmittels mit dem aufgebrachten Zugband aufweist. Die große Dicke reduziert den Tragekomfort und wird als ästhetisch nachteilig angesehen. US 5 185 000 zeigt ein Stützmittel gemäß dem Oberbegriff des Anspruchs 1.

### Aufgabe und Lösung

Aufgabe der Erfindung ist es daher, ein gattungsgemäßes orthopädisches Stützmittel bzw. ein Verfahren zu dessen Herstellung dahingehend weiterzubilden, dass die Elastizität des Stützmittels in elastischen Teilbereichen des Hauptkörpers zur Erzielung einer vorteilhaften Kompressions-, Korrektur- und Stützfunktion gezielt verminderbar ist, ohne dass dies dazu führt, dass das Stützmittel unangenehm zu tragen ist bzw. eine ungewünscht große Dicke aufweist.

Erfindungsgemäß ist zu diesem Zweck vorgesehen, dass zwischen einer Innenseite und einer Außenseite des Hauptkörpers mindestens ein Aufnahmekanal vorgesehen ist, der sich zumindest auch durch elastische Teilbereiche des Hauptkörpers erstreckt, dass darüber hinaus der Aufnahmekanal zur Innenseite hin und zur Außenseite hin durch mindestens einen Strickfaden der Maschenware sowie vorzugsweise durch zusätzliche Schussfäden und/oder Kettfäden der Maschenware begrenzt wird und das weiterhin in den Aufnahmekanal ein unelastisches Stützband vorzugsweise gestreckt eingelegt ist, welches nicht an der Maschenbildung der Maschenware beteiligt ist.

Der Hauptkörper des orthopädischen Stützmittels ist dafür ausgebildet, dass zu stützende Körperteil des Patienten, beispielsweise also ein Kniegelenk oder ein Sprunggelenk, zu umgeben und mit einer Innenseite am Körper des Patienten anzuliegen. Der Hauptkörper umgibt das entsprechende Körperteil zumindest überwiegend, wobei es jedoch nicht erforderlich ist, dass der Hauptkörper beispielsweise als Rundgestrick ausgebildet ist, um das Körperteil vollständig zu umgeben. Stattdessen kann auch vorgesehen sein, dass Teilabschnitte des Stützmittels durch vom Hauptkörper separate Elemente wie beispielsweise durch einen textilen Verbindungsabschnitt oder durch einen Reißverschluss zur Verbindung gegenüberliegender Kanten des Hauptkörpers gebildet werden. Der Hauptkörper ist als Maschenware ausgebildet und verfügt daher über zumindest einen maschenbildenden Strickfaden, der unelastisch ausgebildet sein kann. Zusätzlich zu dem einen oder den mehreren Strickfäden kann die Maschenware auch über entlang der Maschenreihen verlaufende Schussfäden und/oder entlang der Maschenstäbchen verlaufende Kettfäden verfügen, die weitgehend gestreckt verlaufen und die daher zur Aufrechterhaltung der Elastizität zumindest in den elastischen Teilbereichen und zumindest, soweit sie in eine Richtung verlaufen, in der Elastizität gegeben sein soll, elastisch dehnbar ausgebildet sind. Die Maschenware, die den Hauptkörper bildet, erhält ihre Elastizität somit dadurch, dass die maschenbildenden Strickfäden aufgrund der Verformbarkeit der Maschen selbst eine elastische Verformung gestatten und dass die gegebenenfalls vorhandenen Kett- und/oder Schussfäden als solche elastisch ausgebildet sind. Bei gattungsgemäßen sowie erfindungsgemäßen Stützmitteln wird es als bevorzugt angesehen, wenn keine Kettfäden, sondern nur Strickfäden und gegebenenfalls elastische Schussfäden Verwendung finden.

Zusätzlich zu den Strickfäden, Schussfäden und/oder Kettfäden ist erfindungsgemäß unmittelbar im Hauptkörper auch das unelastische Stützband vorgesehen, welches in einem Aufnahmekanal liegt, der sowohl zur Innenseite des Stützmittels hin als auch zur Außenseite des Stützmittels hin durch die oben genannten Strickfäden, Schussfäden und/oder Kettfäden begrenzt ist. Der Aufnahmekanal und das darin liegende gestreckte unelastische Stützband verlaufen im Hauptkörper entlang eines Pfades, bezüglich dessen eine elastische Dehnung zumindest im angelegten Zustand des orthopädischen Stützmittels nicht mehr gewünscht ist. Unter der Unelastizität des Stützbandes wird im Zusammenhang mit der Erfindung insbesondere verstanden, dass das Stützband eine geringere Elastizität als die das Stützband umgebende Maschenware aufweist, so dass die elastische Dehnbarkeit der Maschenware in Erstreckungsrichtung des Stützbandes durch das Stützband gesenkt wird. Als besonders vorteilhaft wird ein Stützband angesehen, welches eine Reißdehnung von weniger als 20%, insbesondere von weniger als 10% aufweist. Insbesondere kann als Stützband auch ein einzelner Garn, vorzugsweise ein hochfester Garn mit vorzugsweise über 50cN/tex Verwendung finden. Das Stützband, insbesondere der genannte hochfeste Garn, weist eine ausreichende Glätte auf, um innerhalb des Aufnahmekanals beweglich zu sein.

Durch die Anordnung des Stützbandes unmittelbar im Hauptkörper des orthopädischen Stützmittels ist das Stützband von außen kaum zu erkennen und somit ästhetisch vorteilhaft untergebracht. Des Weiteren kann durch die Nutzung des Strickfadens und gegebenenfalls der Schussfäden und Kettfäden zur Bildung des Aufnahmekanals auf zusätzliche am Hauptkörper angebrachte Führungsmittel und dafür vorgesehene Applikationen verzichtet werden, so dass eine besonders dünnwandige Gestaltung des Stützmittels realisierbar ist. Die Anordnung des Stützbandes im Hauptkörper führt dabei auch zu einer besonders unmittelbaren elastizitätsvermindernden Wirkung des Stützbandes, da sich der Hauptkörper sowohl innenseitig als auch außenseitig des eingebetteten Stützbandes erstreckt. Von besonderem Vorteil ist darüber hinaus, dass die Einbringung des Stützbandes bereits während der Herstellung des Hauptkörpers möglich ist, so dass in einem einstufigen Herstellungsprozess gleichzeitig die den Hauptkörper bildende Maschenware hergestellt und das Stützband eingelegt werden kann. Eine komplizierte und aufwendige nachträgliche Einbringung des Stützbandes nach Herstellung der Maschenware kann somit vermieden werden.

Die elastizitätsvermindernde Wirkung des Stützbandes wird insbesondere genutzt, um die Gelenkfunktion eines Wirbelsäulengelenks oder eines Gelenks an den Extremitäten eines Patienten zu stützen oder hinsichtlich der Bewegbarkeit einzuschränken. Daneben kann das Stützband auch der Erzielung einer Kompressionswirkung dienen.

Das erfindungsgemäße Stützmittel kann so ausgebildet sein, dass das Stützband entlang der Maschenstäbchen oder alternativ entlang der Maschenreihen verläuft. Eine solche geradlinige Erstreckung kann vor allem bei Bandagen zweckmäßig sein, bei denen es primär auf die Kompressionswirkung ankommt, beispielsweise bei Rückenbandagen. In einem solchen Fall wird es als besonders vorteilhaft angesehen, wenn die im Weiteren noch erläuterten durch den Patienten handhabbaren Festlegungsmittel am Stützband vorgesehen sind.

Insbesondere bei Bandagen, bei denen die Stützwirkung während einer Gelenkbewegung im Vordergrund steht, wie beispielsweise bei Kniebandagen zur Stützung der Kniescheibe, wird es als bevorzugt angesehen, wenn der Aufnahmekanal und das daran eingelegte Stützband zumindest abschnittsweise nichtparallel zu den Maschenstäbchen und zumindest abschnittsweise nichtparallel zu den Maschenreihen der Maschenware verlaufen. Das Stützband kann durch einen derartigen maschenreihen- und maschenstäbchenübergreifenden Verlauf besonders gut an die orthopädischen Anforderungen hinsichtlich der Elastizitätsverminderung angepasst werden. Da die Reibung zwischen dem Stützband einerseits und dem Strickfaden sowie gegebenenfalls den Schussfäden und/oder Kettfäden der Maschenware andererseits möglichst gering gehalten werden sollte, ist es jedoch bevorzugt, wenn zumindest über den überwiegenden Teil der Länge des Stützbandes dieses parallel zu den Maschenreihen und/oder parallel zu den Maschenstäbchen verläuft. Bei den genannten Bandagen, die insbesondere eine Stützwirkung bei der Bewegung entfalten sollen, sind die im Weiteren noch erläuterten durch den Patienten handhabbaren Festlegungsmittel eher entbehrlich als bei den insbesondere eine Kompressionswirkung entfaltenden Bandagen.

Die Maschenware weist eine Maschenwarenbahn auf, wobei der mindestens eine Strickfaden, die Schussfäden und/oder die Kettfäden dieser Maschenwarenbahn gemeinsam den Aufnahmekanal in Richtung der Innenseite und in Richtung der Außenseite begrenzen. Bei einer solchen Gestaltung sind somit die Fäden ein und derselben Maschenwarenbahn ausreichend, um den Aufnahmekanal vollständig zu begrenzen und somit das darin eingelegten Stützband zu führen. Besonders bevorzugt ist bei einer solchen Gestaltung, wenn zumindest die Schussfäden oder die Kettfäden den Aufnahmekanal nach innen oder nach außen begrenzen und wenn zumindest der mindestens eine Strickfaden den Aufnahmekanal in entgegengesetzter Richtung begrenzt.

Bei einer nicht beanspruchten alternativen Gestaltung weist die Maschenware mindestens zwei getrennte Maschenwarenbahnen auf, die zur Bildung des Aufnahmekanals stellenweise miteinander verbunden sind, wobei der Aufnahmekanal in Richtung der Innenseite durch einen Strickfaden, Schussfaden und/oder Kettfaden der ersten Maschenwarenbahn begrenzt wird und wobei der Aufnahmekanal in Richtung der Außenseite durch einen Strickfaden, Schussfaden und/oder Kettfaden der zweiten Maschenwarenbahn begrenzt wird. Bei einer solchen Gestaltung sind der Aufnahmekanal und somit auch das in den Aufnahmekanal eingelegte Stützband nach innen und nach außen jeweils durch Fäden verschiedener Maschenwarenbahnen begrenzt. Die Maschenwarenbahnen sind dabei deckungsgleich und erstrecken sich somit beide über die gesamte Fläche des Hauptkörpers des orthopädischen Stützmittels. Die Verbindung der Maschenwarenbahnen miteinander kann durch eine dritte Maschenwarenbahn erfolgen, durch die Schussfäden , Kettfäden oder insbesondere die Strickfäden einer der beiden Maschenwarenbahnen oder aber durch zusätzliche Verbindungselemente. Hierbei können insbesondere auch alle aus der DE 44 19 985 A1 bekannten Gestaltungen eines Mehrlagengestricks hinsichtlich der Zahl und der Verbindung der Gestrickbahnen Verwendung finden. Auch eine Maschenware mit mindestens zwei getrennten Maschenwarenbahnen kann in einem einheitlichen kontinuierlichen Herstellungsvorgang hergestellt werden, bei dem vorzugsweise auch das Stützband eingelegt wird.

Selbstverständlich ist es auch bei einer Gestaltung mit mehreren getrennten Maschenwarenbahnen möglich, dass der Aufnahmekanal alleine durch Fäden einer der Maschenwarenbahnen, insbesondere der äußeren Maschenwarenbahn, gebildet wird.

Hinsichtlich der beiden Enden des Stützbandes werden verschiedene Varianten zu deren Fixierung als vorteilhaft angesehen. Bei einer ersten Gestaltung sind die beiden Enden des Stützbandes miteinander verbunden, wobei für ein derartiges geschlossenes Stützband vorzugsweise ein umlaufend geschlossener Aufnahmekanal vorgesehen ist. Bei demgegenüber bevorzugten Gestaltungen sind die beiden Enden des Stützbandes an verschiedenen Stellen des Hauptkörpers vorgesehen, wobei eine besonders einfache Gestaltung vorsieht, dass beide Enden fest mit dem Hauptkörper verbunden, beispielsweise mit der Maschenware verknotet sind. Bevorzugt ist es jedoch, wenn eines oder beide Enden mittels eines durch den Patienten handhabbaren Festlegungsmittels am Hauptkörper festgelegt und vom Hauptkörper gelöst werden können. Eine solche Variante mit einem durch den Patienten handhabbaren Festlegungsmittel erlaubt es, die elastizitätsvermindernde Wirkung des Stützbandes erst eintreten zu lassen, wenn das orthopädische Stützmittel bereits angelegt wurde. Das Anlegen des Stützmittels ist in einem solchen Falle daher ungehindert vom Stützband möglich. Ein solches durch den Patienten handhabbares Festlegungsmittel ist vorzugsweise an einem Ende des Stützbandes vorgesehen, welches durch die Außenseite des Hauptkörpers herausgeführt ist. Hierfür ragt vorzugsweise ein mindestens 1 bis 2 cm langer Endabschnitt des Stützbandes aus dem Hauptkörper heraus. An diesem Endabschnitt ist das Festlegungsmittel befestigt.

Das Festlegungsmittel ist vorzugsweise dafür ausgebildet, in variablen Positionen am Hauptkörper befestigt werden zu können. Hierfür können mehrere variable Positionen vorgegeben sein, beispielsweise indem das Festlegungsmittel durch Haken und Ösen am Endabschnitt des Stützbandes bzw. am Hauptkörper gebildet wird. Besonders von Vorteil ist es jedoch, wenn das Festlegungsmittel eine vollständig stufenlos lageflexible Anbringung am Hauptkörper gestattet, beispielsweise indem es durch eine erste Klettfläche am Hauptkörper und eine korrespondierende Klettfläche am Ende des Stützbandes gebildet wird, wobei die Klettflächen in verschiedenen Relativstellungen aneinander anbringbar sind.

Die bisherigen Erläuterungen beziehen sich jeweils auf eine Gestaltung mit einem einzelnen Stützband, aber auch mit mehreren Stützbändern. Insbesondere wenn besonders dünne Stützbänder, beispielsweise hochfeste Garne, Verwendung finden sollen, wird es als bevorzugt angesehen, wenn mehrere Aufnahmekanäle vorgesehen sind, in die jeweils separate Stützbänder eingelegt sind, wobei die Aufnahmekanäle vorzugsweise im Wesentlichen parallel zueinander verlaufen. Neben der Tatsache, dass somit auch bei der Verwendung dünner Stützbänder und Garne eine besonders hohe Reißfestigkeit und eine besonders geringe Elastizität erreicht werden kann, erlaubt die Verwendung mehrerer Stützbänder auch eine eher flächige Stützwirkung als eine Gestaltung, bei der nur ein Stützband Verwendung findet. Als im Wesentlichen paralleler Verlauf der Aufnahmekanäle und Stützbänder wird ein Verlauf angesehen, bei dem sich die Aufnahmekanäle insbesondere nicht überkreuzen. Besonders von Vorteil ist es, wenn die Aufnahmekanäle zwischen 2mm und 20mm voneinander beabstandet sind.

Im Falle einer Gestaltung mit mehreren Stützbändern, die in jeweils eigene Aufnahmekanäle eingelegt sind, wird es als vorteilhaft angesehen, wenn die Stützbänder einen gemeinsamen Festlegungsabschnitt als Teil des Festlegungsmittels aufweisen, der zur durch den Patienten durchzuführenden Festlegung am Hauptkörper vorgesehen ist.

Weiterhin wird es als vorteilhaft angesehen, wenn am Hauptkörper ein gegenüber der Maschenware steiferer Stützkörper wie eine Stützpelotte im Bereich des mindestens einen Aufnahmekanals angebracht ist. Eine Gestaltung mit Stützkörper oder Stützpelotte erlaubt eine besonders wirksame Stützwirkung durch das Stützband. Es wird eine größerflächige Druckverteilung gewährleistet, durch die durch das Stützband ohne Stützkörper bewirkte Druckstellen verhindert werden können. So ist beispielsweise bei einer Gestaltung des Stützmittels als Kniebandage eine solche Pelotte zur flächigen Kraftaufbringung an der Kniescheibe des Patienten von Vorteil. Der Stützkörper kann vorzugsweise als Silikonpolster ausgebildet sein. Die Anbringung erfolgt vorzugsweise überlappend zum Aufnahmekanal des Stützbandes oder zwischen mehreren Aufnahmekanälen.

Verschiedene Typen orthopädischer Stützmittel werden insbesondere als zweckmäßig im Zusammenhang mit der vorliegenden Erfindung angesehen.

Bei einer ersten bevorzugten Gestaltung ist das orthopädische Stützmittel als Kniebandage ausgebildet, wobei der Aufnahmekanal und das Stützband sich innerhalb des Hauptkörpers von einer Beininnenseite kommend beinaußenseitig um einen Kniescheibenbereich herum und weiter bis zur Beininnenseite erstrecken, um die Verlagerung der Kniescheibe nach außen beim Beugen des Knies zu begrenzen. Bei einer solchen Gestaltung wird durch das Stützband im Aufnahmekanal somit erreicht, dass der vorzugsweise mit einem Stützkörper oder einer Stützpelotte ausgebildete Kniescheibenbereich des Hauptkörpers nur in sehr begrenztem Maße beim Beugen des Knies nach außen verlagert wird, da aufgrund der Unelastizität des Stützbandes der Kniescheibenbereich des Hauptkörpers kaum nach außen ausgelenkt werden kann. Dies verhindert oder begrenzt beim Beugen des Knies die orthopädisch ungewünschte Verlagerung der Kniescheibe nach außen.

Bei einer zweiten Variante ist das Stützmittel als Fußbandage ausgebildet, wobei sich der Aufnahmekanal von einem Fußoberseitenbereich zu einem Sprunggelenksbereich erstreckt, um ein Absinken des Fußes zu begrenzen. Dies ist beispielsweise im Falle einer Pereneuslähmung zweckmäßig.

Eine weitere Variante sieht vor, dass das Stützmittel als Fußbandage ausgebildet ist, wobei sich der Aufnahmekanal und das Stützband von einem linksseitigen Sprunggelenksbereich über einen Fersenbereich unterhalb der Ferse des Fußes zu einem rechtsseitigen Sprunggelenksbereich erstrecken. Eine solche Gestaltung, bei der das Stützband etwa U-förmig von der linken Seite des Fußes unter der Ferse hindurch auf der rechten Seite des Fußes verläuft, verhindert wirksam ein Umknicken des Fußes nach innen oder nach außen. Ein derartiges orthopädisches Stützmittel kann insbesondere als Sportbandage ausgebildet sein.

Eine weitere Variante sieht vor, dass das Stützmittel als Hüftbandage ausgebildet ist, wobei sich der Aufnahmekanal und das Stützband von einer Vorderseite des Stützmittels über den Rückenbereich wieder zur Vorderseite erstrecken. Bei einer solchen Gestaltung sind insbesondere die Kompressionswirkung und die Stabilisierungswirkung, die durch das Stützband hervorgerufen werden, von Vorteil.

Die Erfindung betrifft weiterhin auch ein Verfahren zur Herstellung eines orthopädischen Stützmittels nach einem der vorstehenden Ansprüche. Dabei ist erfindungsgemäß vorgesehen, dass das Stützband während des Herstellungsvorgangs der Maschenware kontinuierlich in den während des Herstellungsvorgangs gebildeten Aufnahmekanal eingelegt wird. Bei einem solchen Verfahren wird der Aufnahmekanal gleichsam dadurch gebildet, dass das Stützband kontinuierlich während der Herstellung der Maschenreihen der Maschenware zwischen dem Strickfaden und eventuell vorgesehenen Schussfäden und Kettfäden der Maschenware positioniert wird. Nach Abschluss des Herstellungsvorgangs der Maschenware ist das Stützband somit bereits in den Aufnahmekanal eingelegt. Es bedarf anschließend nur noch etwaiger Maßnahmen zur Festlegung der Enden des Stützbandes am Hauptkörper des Stützmittels, am jeweils anderen Ende des Stützbandes oder aber an einem Festlegungsabschnitt wie beispielsweise einem Klettabschnitt, der zur durch den Patienten durchzuführenden Festlegung am Hauptkörper des Stützmittels vorgesehen ist.

### Kurzbeschreibung der Zeichnungen

Weitere Vorteile und Aspekte der Erfindung ergeben sich außer aus den Ansprüchen auch aus der nachfolgenden Beschreibung von Ausführungsbeispielen der Erfindung, die anhand der Figuren erläutert sind. Dabei zeigen:
- Fig. 1a und 1b: eine erste Ausführungsform eines erfindungsgemäßen orthopädischen Stützmittels,
- Fig. 2: eine zweite Ausführungsform eines erfindungsgemä- ßen orthopädischen Stützmittels,
- Fig. 3: eine dritte Ausführungsform eines erfindungsgemäßen orthopädischen Stützmittels und
- Fig. 4: eine schematische Darstellung der Anordnung des unelastischen Stützbandes in der Maschenware der orthopädischen Stützmittel gemäß der Fig. 1 bis 3.

### Detaillierte Beschreibung der Ausführungsbeispiele

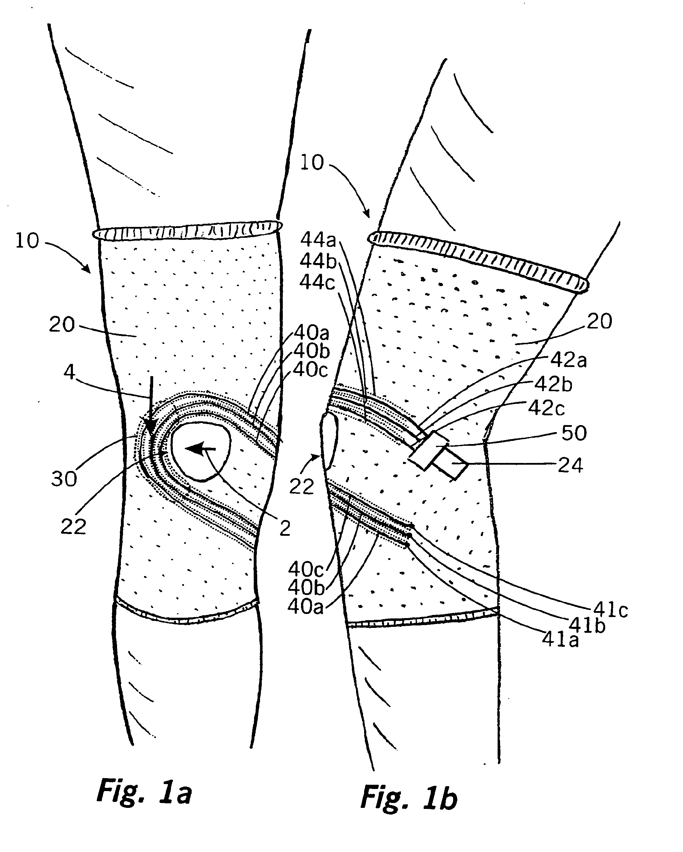

Die Fig. 1a und 1b zeigen ein erstes orthopädisches Stützmittel gemäß der vorliegenden Erfindung. Dieses ist als Kniebandage 10 für ein rechtes Bein ausgebildet und in der Darstellung der Fig. 1a in einer Frontansicht von vorne und in der Darstellung der Fig. 1b in einer Seitenansicht von links dargestellt.

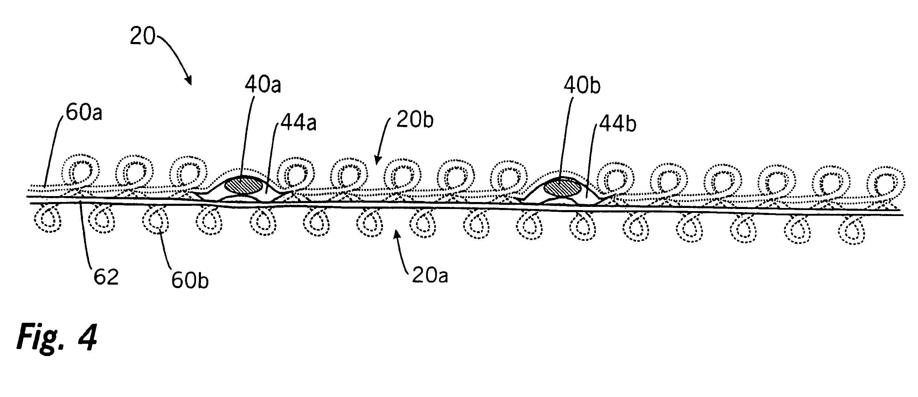

Die Kniebandage 10 weist einen Hauptkörper 20 auf, der in im Zusammenhang mit der Fig. 4 noch nachfolgend beschriebener Weise als in sich elastische Maschenware ausgebildet ist. In einem Kniescheibenbereich 22 des Hauptkörpers 20 ist an diesem ein in etwa mondsichelförmiger Silikon-Stützkörper 30 innenseitig befestigt, der aufgrund seiner an sich in der Fig. 1a nicht erkennbaren Lage an der Innenseite des Hauptkörpers 20 gestrichelt dargestellt ist. Dieser Stützkörper 30 liegt an der aus der Perspektive des Patienten rechten Seite der Kniescheibe des Patienten an.

Um zu verhindern, dass bei einem Beugen des Knies aufgrund der Elastizität des Hauptkörpers 20 der Kniescheibenbereich 22 des Hauptkörpers 20 gemeinsam mit dem Stützkörper 30 in Richtung des Pfeils 2 in erheblichem Maße nach außen gedrückt wird, sind insgesamt drei zueinander parallel verlaufende und als hochfeste Garne ausgebildete Stützbänder 40a, 40b, 40c vorgesehen, die sich von einer Beininnenseite des Hauptkörpers 20 außen um den Kniescheibenbereich 22 herum bis wieder zur Beininnenseite erstrecken. Diese Stützbänder 40a, 40b, 40c sind zu einem überwiegenden Anteil in gepunktet dargestellten Aufnahmekanälen 44a, 44b, 44c zwischen eine Innenoberfläche und einer Außenoberfläche des Hauptkörpers 20 angeordnet und daher von außen kaum zu erkennen. Die hochfesten Garne, die die Stützbänder 40a, 40b, 40c bilden, weisen eine maximale Reißdehnung von 30% und eine Festigkeit von etwa 58 cN/tex auf.

Die unteren Enden 41a, 41b, 41c der drei Stützbänder 40a, 40b, 40c sind in der aus Fig. 1b ersichtlichen Weise mittels Knoten an definierten Stellen des Hauptkörpers 20 befestigt. Die gegenüberliegenden oberen Enden 42a, 42b, 42c sind an den Enden der Aufnahmekanäle 44a, 44b, 44c durch die Außenoberfläche des Hauptkörpers 20 hindurch nach außen geführt und dort mit einem gemeinsamen Festlegungsabschnitt 50 verbunden, an dessen in Fig. 1b nicht dargestellter Unterseite ein Klettabschnitt vorgesehen ist. Korrespondierend zu diesem Klettabschnitt des Festlegungsabschnitts 50 ist an der Außenoberfläche des Hauptkörpers 20 eine Klettfläche 24 vorgesehen, die die Festlegung der Enden 42a, 42b, 42c in einer durch den Patienten festzulegenden individuellen Lage gestattet.

Durch die Stützbänder 40a, 40b, 40c ist es möglich, die Elastizität des Hauptkörpers 20 gezielt bezogen auf deren Erstreckungsrichtung zu vermindern. Da die Stützbänder 40a, 40b, 40c eine geringere Elastizität als die Maschenware des Hauptkörpers 20 aufweisen, ist eine Dehnung des Hauptkörpers entlang des Verlaufs der Stützbänder nicht mehr oder nur noch in geringfügigem Maße möglich, sobald der Festlegungsabschnitt 50 mit der Klettfläche 24 verbunden wurde. Somit kann nach einem durch die Stützbänder 40a, 40 b, 40c nicht beeinträchtigten Anlegen der Bandage 10 durch Festlegen des Festlegungsabschnitts 50 die Elastizität des Hauptkörpers 20 im Kniescheibenbereich 22 herabgesetzt werden, so dass dieser Kniescheibenbereich 22 und der Stützkörper 30 bei der nachfolgenden Nutzung eine Verlagerung der Kniescheibe des Patienten nach außen wirksam begrenzen.

Bei der Kniebandage der Fig. 1a und 1b sind die Festlegungsmittel 50, 24 insbesondere als Erleichterung während des Anlegens der Bandage gedacht. Alternative Ausführungsformen können daher auch Stützbänder aufweisen, die an beiden Enden mit dem Hauptkörper fest verbunden sind. In Hinblick auf die Stützfunktion ergibt sich hierdurch keine wesentliche Veränderung.

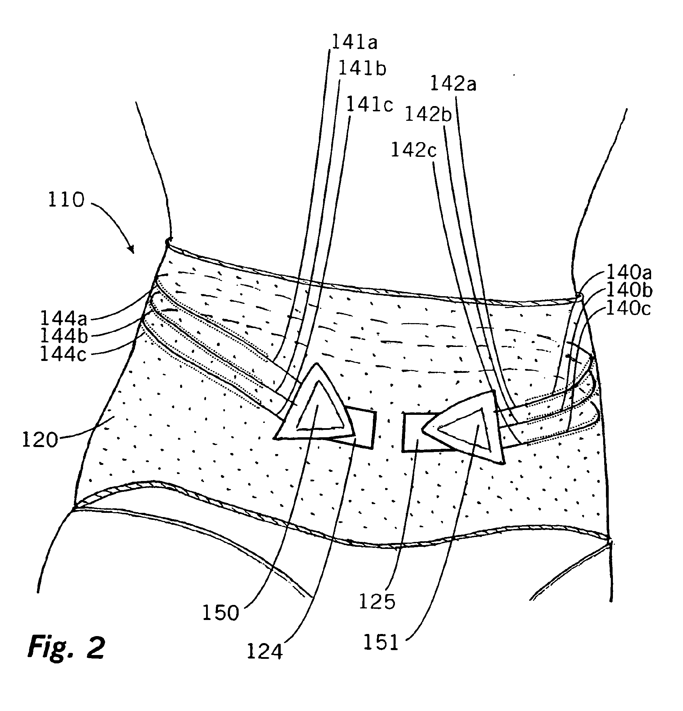

Fig. 2 zeigt eine Hüftbandage 110, an der ebenfalls drei zueinander parallele unelastische Stützbänder 140a, 140b, 140c vorgesehen sind. Diese Stützbänder 140a, 140b, 140c sind wie auch bei der Kniebandage der Fig. 1a und 1b in Aufnahmekanäle 144a, 144b, 144c aufgenommen, die zwischen einer Außenseite und einer Innenseite eines als Maschenware ausgebildeten Hauptkörpers 120 der Hüftbandage 110 angeordnet sind. Abweichend von der Gestaltung der Fig. 1 sind bei dieser Hüftbandage 110 jeweils beide Enden 141a, 141b, 141c, 142a, 142b, 142c mit Festlegungsabschnitten 150, 151 versehen, die mittels hauptkörperseitiger Klettflächen 124, 125 in flexibler Position am Hauptkörper 120 festgelegt werden können. Wie auch bei der Kniebandage der Fig. 1 wird auch bei dieser Hüftbandage während des Anlegens mindestens einer der Festlegungsabschnitte 150, 151 von der korrespondierenden Klettfläche 124, 125 gelöst, um die elastizitätsvermindernde bzw. elastizitätsvermeidende Wirkung der Stützbänder 140a, 140b, 140c während des Anlegens der Hüftbandage nicht eintreten zu lassen. Nach dem Anlegen der Hüftbandage 110 kann die elastizitätsvermindernde Wirkung dann durch die Festlegung dieses Festlegungsabschnitts 150, 151 individuell angepasst hergestellt werden und somit die Kompressionswirkung und Stabilisierungswirkung der Hüftbandage 120 gezielt beeinflusst werden.

Bei der Hüftbandage 110 der Fig. 2 ist der Hauptkörper 120 bei nicht festgelegten Festlegungsabschnitten 150, 151 in Erstreckungsrichtung der Aufnahmekanäle 140a, 140b, 140c elastisch ausgebildet, um die gewünschte Kompressionswirkung zu entfalten. Bei einer solchen Bandage ist die durch die Festlegungsabschnitte 150, 151 erzielbare Einstellbarkeit als örtlich begrenzte Unterstützung der Kompressionswirkung gedacht, wobei die jeweils abseits der Aufnahmekanäle angeordneten Bereiche des Hauptkörper 120 auch nach Festlegen der Festlegungsabschnitte 150, 151 unverändert eine parallel zur Erstreckungsrichtung der Aufnahmekanäle 144a, 144b, 144c wirkende Elastizität behalten, die durch elastische Schussfäden oder Kettfäden erzielt werden kann und die durch die Stützbänder nicht beeinträchtigt wird. Es wird lediglich partiell die maximale Dehnung des Hauptkörpers 120 begrenzt.

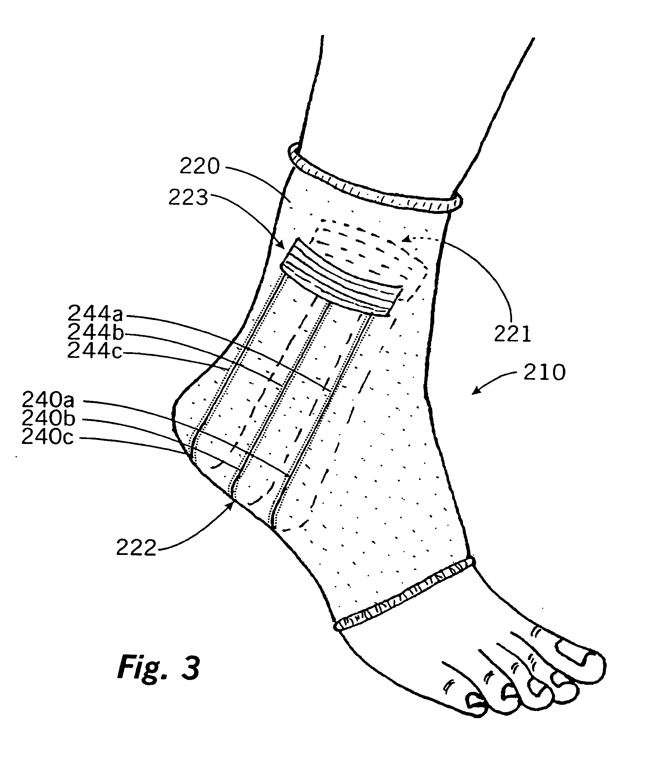

Bei der Ausgestaltung der Fig. 3 handelt es sich um eine als Sportbandage ausgebildete Sprunggelenksbandage 210. Wie auch die vorangegangenen Bandagen weist diese Sprunggelenksbandage 210 einen als Maschenware ausgebildeten und elastischen Hauptkörper 220 auf, der im Falle der Bandage der Fig. 3 in etwa die Form eines zehenlosen Sockens hat. Zwischen der Innenseite und der Außenseite dieses Hauptkörpers 220 sind wiederum Aufnahmekanäle 244a, 244b, 244c und darin eingelegte Stützbänder 240a, 240b, 240c vorgesehen. Diese erstrecken sich von einer linken Fußseite 221 des Hauptkörpers 220 über einen Fersenbereich 222 des Hauptkörpers 220 zu einer rechten Seite 223 des Hauptkörpers 220. Anders als bei den vorangegangenen Ausführungsformen sind dabei jeweils beide Enden 241 a, 241 b, 241 c, 242a, 242b, 242c an festen Stellen des Hauptkörpers 220 mit diesem vernäht. Die Bandage der Fig. 3 verhindert ein Umknicken des Fußes.

Wie bereits erwähnt, sind die Stützbänder 40a, 40b, 40c, 140a, 140b, 140c, 240a, 240b, 240c der Bandagen 10, 110, 210 der Fig. 1a, 1b, 2, 3 jeweils zwischen einer Innenseite und einer Außenseite des jeweiligen Hauptkörpers 20, 120, 220 angeordnet. Fig. 4 erläutert dies anhand einer Darstellung der Maschenware, die den Hauptkörper 20 der Bandage 10 der Fig. 1a und 1b zeigt. Der Pfeil 4 in Fig. 1a verdeutlicht die Blickrichtung, die der Darstellung der Fig. 4 zugrunde liegt. Fig. 4 zeigt einen Ausschnitt, der nur zwei der drei Stützbänder 40a, 40b, 40c umfasst.

Die Maschenware des Hauptkörpers 20 wird in an sich gängiger Art und Weise aus zwei Strickfäden 60a, 60b gebildet, die in Fig. 4 gepunktet und gestrichelt dargestellt sind. Diese Strickfäden 60a, 60b bilden die für Maschenware charakteristischen Maschen. Neben den Strickfäden 60a, 60b sind längs gestreckte Schussfäden 62 vorgesehen, die sich bezogen auf die Darstellung der Fig. 1a und 1b im Wesentlichen horizontal in der Maschenware des Hauptkörpers 20 erstrecken.

Die Stützbänder 40a, 40b sind wie bereits erwähnt in Aufnahmekanälen 44a, 44b angeordnet, die in Richtung einer Innenseite 20a des Hauptkörpers 20 unter anderem durch die Schussfäden 62 begrenzt werden und die in Richtung einer Außenseite 20b des Hauptkörpers 20 insbesondere durch den Strickfaden 60a begrenzt werden. Zur Definierung der Aufnahmekanäle 44a, 44b bedarf es somit keiner Elemente, die nicht unmittelbar Teil der Maschenware sind.

Die Herstellung der Maschenware der Fig. 4 erfolgt in nicht näher dargestellten Art und Weise zeitgleich mit der Einbringung der Stützbänder 40a, 40b, 40c. Während durch eine Strickmaschine in an sich bekannter Weise die Maschenreihen nacheinander hergestellt werden, werden durch insgesamt drei separate Fadenführer die Stützbänder 40a, 40b, 40c in die entstehende Maschenware eingelegt, so dass sich der Aufbau der Fig. 4 ergibt.

Die die Hauptkörper 120, 220 der Ausführungsformen der Fig. 2 und 3 bildende Maschenware kann entsprechend der Darstellung der Fig. 4 aufgebaut sein. Eine nicht dargestellte Alternative weist zwei getrennte Maschenwarenbahnen mit jeweils eigenem Strickfaden auf, wobei die Strickfäden der beiden Maschenwarenbahnen miteinander nicht unmittelbar verbunden sind, sondern durch separate Bindungselemente wie beispielsweise gemeinsame Schussfäden oder Kettfäden verbunden sind. In einem solchen Fall können die Aufnahmekanäle 44a, 44b, 44c, 144a, 144b, 144c, 244a, 244b, 244c zwischen dem Maschenwarenbahnen vorgesehen sein und durch die Strickfäden der beiden Maschenwarenbahnen nach innen und nach außen begrenzt sein. Auch ein daraus resultierender mehrlagiger Hauptkörper kann in einem kontinuierlichen Verfahren hergestellt werden, bei dem die Stützbänder 40a, 40b, 40c, 140a, 140b, 140c, 240a, 240b, 240c während der gemeinsamen Herstellung der Lagen und deren Verbindung in den Hauptkörper kontinuierlich eingebracht werden.

## Patentansprüche

1. Orthopädisches Stützmittel (10; 110; 210), insbesondere in Form einer Bandage (10; 110; 210), mit einem formflexiblen, als Maschenware ausgebildeten und zumindest in elastischen Teilbereichen elastischen Hauptkörper (20; 120; 220), der dafür ausgebildet ist, ein zu stützendes Körperteil eines Patienten zu umgeben, wobei
- zwischen einer Innenseite (20a) und einer Außenseite (20b) des Hauptkörpers (20; 120; 220) mindestens ein Aufnahmekanal (44a, 44b, 44c; 144a, 144b, 144c; 244a, 244b, 244c) vorgesehen ist, der sich zumindest auch durch elastische Teilbereiche des Hauptkörpers (20; 120; 220) erstreckt,
- der Aufnahmekanal zur Innenseite hin und zur Außenseite hin durch mindestens einen Strickfaden (60a, 60b) der Maschenware und vorzugsweise durch Schussfäden (62) und/oder Kettfäden der Maschenware begrenzt wird und
- in den Aufnahmekanal ein unelastisches Stützband (40a, 40b, 40c; 140a, 140b, 140c; 240a, 240b, 240c) eingelegt ist, welches nicht an der Maschenbildung der Maschenware des Hauptkörpers (20; 120; 220) beteiligt ist,
**dadurch gekennzeichnet, dass**
die Maschenware eine Maschenwarenbahn umfasst, wobei der mindestens eine Strickfaden (60a, 60b), die Schussfäden (62) und/oder die Kettfäden dieser Maschenwarenbahn den Aufnahmekanal (44a, 44b, 44c; 144a, 144b, 144c; 244a, 244b, 244c) in Richtung der Innenseite (20a) und in Richtung der Außenseite (20b) begrenzen.

2. Orthopädisches Stützmittel nach Anspruch 1,
**dadurch gekennzeichnet, dass**
der Aufnahmekanal (44a, 44b, 44c; 144a, 144b, 144c; 244a, 244b, 244c) und das Stützband (40a, 40b, 40c; 140a, 140b, 140c; 240a, 240b, 240c) zumindest abschnittsweise nichtparallel zu den Maschenstäbchen und zumindest abschnittsweise nichtparallel zu den Maschenreihen der Maschenware verlaufen.

3. Orthopädisches Stützmittel nach Anspruch 1 oder 2,
**dadurch gekennzeichnet, dass**
die beiden Enden (41 a, 42a, 41 b, 42b, 41 c, 42c) des Stützbandes
- aneinander befestigt sind oder
- am Bandagenkörper (20) befestigt und/oder mittels eines werkzeuglos handhabbaren Festlegungsmittels (24, 50) befestigbar sind.

4. Orthopädisches Stützmittel nach Anspruch 3,
**dadurch gekennzeichnet, dass**
das Festlegungsmittel (24, 50; 124, 125, 150, 151) zur Festlegung des jeweiligen Endes (41a, 41b, 41c; 141a, 141b, 141c, 142a, 142b, 142c) des Stützbandes (40a, 40b, 40c; 140a, 140b, 140c) in variablen Positionen an der Maschenware ausgebildet ist.

5. Orthopädisches Stützmittel nach einem der vorherigen Ansprüche,
**dadurch gekennzeichnet, dass**
mehrere Aufnahmekanäle (44a, 44b, 44c; 144a, 144b, 144c; 244a, 244b, 244c) vorgesehen sind, in die jeweils separate Stützbänder (40a, 40b, 40c; 140a, 140b, 140c; 240a, 240b, 240c) eingelegt sind, wobei die Aufnahmekanäle (44a, 44b, 44c; 144a, 144b, 144c; 244a, 244b, 244c) vorzugsweise im Wesentlichen parallel zueinander verlaufen.

6. Orthopädisches Stützmittel nach einem der vorherigen Ansprüche,
**dadurch gekennzeichnet, dass**
am Hauptkörper (20) ein gegenüber der Maschenware steiferer Stützkörper (30) im Bereich des Aufnahmekanals (44a, 44b, 44c) angebracht ist.

7. Orthopädisches Stützmittel nach einem der vorherigen Ansprüche,
**dadurch gekennzeichnet, dass**
das unelastische Stützband (40a, 40b, 40c; 140a, 140b, 140c; 240a, 240b, 240c) als unelastischer Faden ausgebildet ist.

8. Orthopädisches Stützmittel nach einem der vorherigen Ansprüche,
**dadurch gekennzeichnet, dass**
das orthopädische Stützmittel als Kniebandage (10) ausgebildet ist, wobei der Aufnahmekanal (44a, 44b, 44c) und das Stützband (40a, 40b, 40c) sich innerhalb des Hauptkörpers (20) von einer Beininnenseite beinaußenseitig um einen Kniescheibenbereich (22) herum und weiter bis zur Beininnenseite erstrecken, um die Verlagerung der Kniescheibe nach außen beim Beugen des Knies zu begrenzen.

9. Orthopädisches Stützmittel nach einem Ansprüche 1 bis 7,
**dadurch gekennzeichnet, dass**
das Stützmittel als Fußbandage ausgebildet ist, wobei sich der Aufnahmekanal und das Stützband von einem Fußoberseitenbereich bis zu einem Sprunggelenksbereich erstrecken, um ein Absinken des Fußes zu begrenzen.

10. Orthopädisches Stützmittel nach einem Ansprüche 1 bis 7,
**dadurch gekennzeichnet, dass**
das Stützmittel als Fußbandage (220) ausgebildet ist, wobei sich der Aufnahmekanal (244a, 244b, 244c) und das Stützband (240a, 240b, 240c) von einem linksseitigen Sprunggelenksbereich (221) über einen Fersenbereich (222) zu einem rechtsseitigen Sprunggelenksbereich (223) erstrecken.

11. Orthopädisches Stützmittel nach einem Ansprüche 1 bis 7,
das Stützmittel als Hüftbandage (120) ausgebildet ist, wobei sich der Aufnahmekanal (144a, 144b, 144c) und das Stützband (140a, 140b, 140c) von einer Vorderseite über den Rückenbereich wieder zur Vorderseite erstrecken.

12. Verfahren zur Herstellung eines orthopädischen Stützmittels nach einem der vorstehenden Ansprüche,
**dadurch gekennzeichnet, dass**
das Stützband (40a, 40b, 40c; 140a, 140b, 140c; 240a, 240b, 240c) während des Herstellungsvorgangs der Maschenware kontinuierlich in den während des Herstellvorgangs gebildeten Aufnahmekanal (44a, 44b, 44c; 144a, 144b, 144c; 244a, 244b, 244c) eingelegt wird.

## Claims

1. Orthopedic aid (10; 110; 210), in particular in form of a bandage (10; 110; 210), with a shape-flexible, as knitted fabric configured and at least in portions elastic main body (20; 120; 220) which is configured to surround a patient's body part to be supported, wherein
- between an inner side (20a) and an outer side (20b) of the main body (20; 120; 220) at least one receiving channel (44a, 44b, 44c; 144a, 144b, 144c; 244a, 244b, 244c) is provided which extends at least also through elastic portions of the main body (20; 120; 220),
- the receiving channel is confined towards the inner side and towards the outer side by at least one knitting thread (60a, 60b) of the knitted fabric and preferably by filling threads (62) and/or warp threads of the knitted fabric and
- an inelastic support strap (40a, 40b, 40c; 140a, 140b, 140c; 240a, 240b, 240c) which is not involved in the formation of meshes of the knitted fabric of the main body (20; 120; 220) is placed in the receiving channel,
**characterized in that** the knitted fabric comprises a knitted fabric panel, wherein the at least one knitting thread (60a, 60b), the filling threads (62) and/or the warp threads of this knitting fabric panel confine the receiving channel (44a, 44b, 44c; 144a, 144b, 144c; 244a, 244b, 244c) in the direction of the inner side (20a) and in the direction of the outer side (20b).

2. Orthopedic aid according to claim 1,
**characterized in that** the receiving channel (44a, 44b, 44c; 144a, 144b, 144c; 244a, 244b, 244c) and the supporting strap (40a, 40b, 40c; 140a, 140b, 140c; 240a, 240b, 240c) run at least in sections non-parallel to the stitch wales and at least in sections non-parallel to the stitch courses of the knitted fabric.

3. Orthopedic aid according to claim 1 or 2,
**characterized in that** the two ends (41a, 42a, 41b, 42b, 41c, 42c) of the supporting strap
- are fixed to each other or
- are fixed at the bandage body (20) and/or are fixable by a tool-less operable fixation means (24, 50).

4. Orthopedic aid according to claim 3,
**characterized in that** the fixation means (24, 50; 124, 125, 150, 151) is configured to fix the respective ends (41a, 41b, 41c; 141a, 141b, 141c, 142a, 142b, 142c) of the supporting strap (40a, 40b, 40c; 140a, 140b, 140c) in variable positions at the knitted fabric.

5. Orthopedic aid according to one of the preceding claims,
**characterized in that** several receiving channels (44a, 44b, 44c; 144a, 144b, 144c; 244a, 244b, 244c) are provided, in each of which separate supporting straps (40a, 40b, 40c; 140a, 140b, 140c; 240a, 240b, 240c) are placed, wherein the receiving channels (44a, 44b, 44c; 144a, 144b, 144c; 244a, 244b, 244c) preferably run substantially parallel to each other.

6. Orthopedic aid according to one of the preceding claims,
**characterized in that** a supporting body (30) being stiffer compared to the knitted fabric is mounted to the main body (20) in the region of the receiving channel (44a, 44b, 44c).

7. Orthopedic aid according to one of the preceding claims,
**characterized in that** the inelastic supporting strap (40a, 40b, 40c; 140a, 140b, 140c; 240a, 240b, 240c) is configured as an inelastic thread.

8. Orthopedic aid according to one of the preceding claims,
**characterized in that** the orthopedic aid is configured as a knee bandage (10), wherein the receiving channel (44a, 44b, 44c) and the supporting strap (40a, 40b, 40c) extend within in the main body (20) from an inner side of the leg around an area of the knee cap at the leg's outer side and further to the inner side of the leg to limit the displacement of the knee cap to the outside during bending of the knee.

9. Orthopedic aid according to one of the claims 1 to 7,
**characterized in that** the orthopedic aid is configured as a as a foot bandage, wherein the receiving channel and the supporting strap extend from an upside of the foot to an area of the ankle to limit a descent of the foot.

10. Orthopedic aid according to one of the claims 1 to 7,
**characterized in that** the orthopedic aid is configured as a foot bandage (220), wherein the receiving channel (244a, 244b, 244c) and the supporting strap (240a, 240b, 240c) extend from a left-hand area of the ankle (221) across an area of the heel (222) to a right-hand area of the ankle (223).

11. Orthopedic aid according to one of the claims 1 to 7,
**characterized in that** the orthopedic aid is configured as a hip bandage (120), wherein the receiving channel (144a, 144b, 144c) and the supporting strap (140a, 140b, 140c) extend from a frontside across the back-area again to the frontside.

12. Method for producing an orthopedic aid according to one of the preceding claims,
**characterized in that** during the production process of the knitted fabric, the supporting strap (40a, 40b, 40c; 140a, 140b, 140c; 240a, 240b, 240c) is continuously placed in the receiving channel (44a, 44b, 44c; 144a, 144b, 144c; 244a, 244b, 244c) formed during the production process.

## Revendications

1. Moyen de support orthopédique (10; 110; 210), se présentant en particulier sous forme de bandage (10; 110 ; 210), avec un corps principal (20; 120; 220) de forme flexible et élastique pour le moins dans des zones partielles élastiques, réalisé en tricot et conçu pour entourer une partie du corps à soutenir d'un patient,
- au moins un canal récepteur (44a, 44b, 44c; 144a, 144b, 144c; 244a, 244b, 244c), qui s'étend pour le moins également à travers des zones partielles élastiques du corps principal (20; 120; 220), étant prévu entre un côté intérieur (20a) et un côté extérieur (20b) dudit corps principal (20; 120; 220),
- le canal récepteur étant délimité vers le côté intérieur et vers le côté extérieur par au moins un fil tricoté (60a, 60b) du tricot, et préférentiellement par des fils de trame (62) et/ou par des fils de chaîne du tricot, et
- une bande de support non élastique (40a, 40b 40c; 140a, 140b, 140c; 240a, 240b, 240c), qui ne contribue pas à la formation des mailles du tricot, étant placée dans le canal récepteur du corps principal (20; 120; 220)
**caractérisé en ce que**
le tricot comprend une bande de tricot, ledit fil de tricot (60a, 60b), les fils de trame (62) et/ou les fils de chaîne de cette bande de tricot délimitant le canal récepteur (44a, 44b, 44c; 144a, 144b, 144c; 244a, 244b, 244c) vers le côté intérieur (20a) et vers le côté extérieur (20b).

2. Moyen de support orthopédique selon la revendication 1,
**caractérisé en ce que**
le canal récepteur (44a, 44b, 44c; 144a, 144b, 144c; 244a, 244b, 244c) et la bande de support (40a, 40b, 40c; 140a, 140b, 140c; 240a, 240b, 240c) passant au moins par endroits de manière non parallèle par rapport aux colonnes de mailles et au moins par endroits de manière non parallèle par rapport aux rangées de mailles.

3. Moyen de support orthopédique selon la revendication 1 ou 2,
**caractérisé en ce que**
les deux extrémités (41a, 42a, 41b, 42b, 41c, 42c) de la bande de support
- sont fixées l'une à l'autre ou
- sont fixées sur le corps de bandage (20) et/ou fixables au moyen d'un moyen de serrage (24, 50) manipulable sans outil.

4. Moyen de support orthopédique selon la revendication 3,
**caractérisé en ce que**
le moyen de serrage (24, 50; 124, 125, 150, 151) destiné au serrage de chacune des extrémités (41a, 41b, 41c; 141a, 141b, 141c, 142a, 142b, 142c) est réalisé dans des positions variables sur le tricot.

5. Moyen de support orthopédique selon l'une des revendications précédentes,
**caractérisé en ce que**
plusieurs canaux récepteurs (44a, 44b, 44c; 144a, 144b, 144c; 244a, 244b, 244c), dans lesquels sont placées respectivement des bandes de support distinctes (40a, 40b, 40c; 140a, 140b, 140c; 240a, 240b, 240c), sont prévus, les canaux récepteurs (44a, 44b, 44c; 144a, 144b, 144c; 244a, 244b, 244c) s'étendant préférentiellement pour l'essentiel de manière parallèle les uns par rapport aux autres.

6. Moyen de support orthopédique selon l'une des revendications précédentes
**caractérisé en ce que**
un corps de support (30) plus rigide que le tricot est appliqué sur le corps principal (20), dans la zone du canal récepteur (44a, 44b, 44c).

7. Moyen de support orthopédique selon l'une des revendications précédentes,
**caractérisé en ce que**
la bande de support non élastique (40a, 40b, 40c; 140a, 140b, 140c; 240a, 240b, 240c) est réalisé sous forme de fil non élastique.

8. Moyen de support orthopédique selon l'une des revendications précédentes,
**caractérisé en ce que**
le moyen de support orthopédique est réalisé sous forme de genouillère (10), le canal récepteur (44a, 44b, 44c) et la bande de support (40a, 40b, 40c) s'étendant, à l'intérieur du corps principal (20), d'un côté intérieur de la jambe vers le côté extérieur de la jambe pour faire le tour d'une zone de rotule (22) et revenir ensuite vers le côté intérieur de la jambe, afin de limiter le déplacement vers l'extérieur de la rotule lors de la flexion du genou.

9. Moyen de support orthopédique selon l'une des revendications 1 à 7, **caractérisé en ce que**
le moyen de support est réalisé sous forme de chevillère, le canal récepteur et la bande de support s'étendant depuis une zone de la face supérieure du pied jusqu'à une zone d'articulation de la cheville afin de limiter le fléchissement du pied vers le bas.

10. Moyen de support orthopédique selon l'une des revendications 1 à 7, **caractérisé en ce que**
le moyen de support est réalisé sous forme de chevillère (220), le canal récepteur (244a, 244b, 244c) et la bande de support (240a, 240b, 240c) s'étendant depuis une zone d'articulation de cheville (221) gauche vers une zone d'articulation de cheville (223) droite, en passant par une zone de talon (222).

11. Moyen de support orthopédique selon l'une des revendications 1 à 7, **caractérisé en ce que**
le moyen de support est réalisé sous forme de hanchière (120), le canal récepteur (144a, 144b, 144c) et la bande de support (140a, 140b, 140c) s'étendant d'une face avant à l'autre face avant, après être passés par une zone dorsale.

12. Moyen de support orthopédique selon l'une des revendications 1 à 7, **caractérisé en ce que**
la bande de support (40a, 40b, 40c; 140a, 140b, 140c; 240a, 240b, 240c) est, pendant la passe de fabrication du tricot, placée de manière continue dans le canal récepteur (44a, 44b, 44c; 144a, 144b, 144c; 244a, 244b, 244c) formé pendant la passe de fabrication.
